Europäisches Patentamt

European Patent Office

Office européen des brevets

(11) Veröffentlichungsnummer: **0 452 849 A1**

## EUROPÄISCHE PATENTANMELDUNG

(21) Anmeldenummer: **91105971.5**

(22) Anmeldetag: **15.04.91**

(51) Int. Cl.5: **C12P 21/08**, C07K 15/06, C07K 3/18, C12N 5/12, G01N 33/577

(30) Priorität: **18.04.90 DE 4012341**

(43) Veröffentlichungstag der Anmeldung:
**23.10.91 Patentblatt 91/43**

(84) Benannte Vertragsstaaten:
**AT BE CH DE DK ES FR GB GR IT LI LU NL SE**

(71) Anmelder: **BEHRINGWERKE Aktiengesellschaft**
**Postfach 1140**
**W-3550 Marburg 1(DE)**

(72) Erfinder: **Hock, Hans, Dr.**
**Haselhecke 8**
**W-3550 Marburg(DE)**
Erfinder: **Römisch, Jürgen, Dr.**
**Am Kähnelplatz 6**
**W-3550 Marburg(DE)**

(74) Vertreter: **Klein, Otto, Dr. et al**
**Hoechst AG Zentrale Patentabteilung**
**Postfach 80 03 20**
**W-6230 Frankfurt am Main 80(DE)**

(54) **Monoklonale Antikörper gegen PP4, Verfahren zu ihrer Herstellung und ihre Verwendung.**

(57) Die Erfindung betrifft monoklonale Antikörper, spezifisch gegen das Protein PP4 gerichtet, solche Antikörper produzierende Hybridoma-Zellinien, Verfahren zur Herstellung solcher Antikörper sowie ihre Verwendung zur Reinigung von PP4 oder zur Diagnose.

EP 0 452 849 A1

Rank Xerox (UK) Business Services

Die Erfindung betrifft monoklonale Antikörper, spezifisch gegen das Protein PP4 gerichtet, solche Antikörper produzierende Hybridoma-Zellinien, sowie ihre Verwendung.

Das Placenta-Protein 4 (PP4) wurde erstmals von Bohn isoliert und physikochemisch charakterisiert (EP 0123 307). Später fanden auch andere Arbeitsgruppen dieses Protein, ohne die Identität zunächst zu erkennen, und bezeichneten es als Annexin V, Lipocortin V oder VAC-alpha. Bei der Sequenzierung der entsprechenden cDNA (Grundmann et al. (1988) Proc. Natl. Acad. Sci. 85, 3708-3712) zeigte sich, daß PP4 einer Familie von Proteinen angehört, die jetzt als Lipocortine oder Annexine bezeichnet werden.

Die Annexine spielen eine entscheidende regulatorische Rolle bei der Freisetzung von Entzündungsmediatoren. Durch Applikation von Annexinen kann die Freisetzung von Entzündungsmediatoren inhibiert werden.

Die Annexine sind wertvolle Therapeutika bei Gerinnungsstörungen wie der disseminierten intravaskulären Gerinnung (DIC).

Zur Gewinnung von PP4 stellt humane Placenta eine geeignete Quelle dar, die in ausreichender Menge verfügbar ist. Die etablierten Reinigungsverfahren für PP4 aus natürlichen Vorkommen sind jedoch aufwendig und mit Verlusten an PP4 verbunden. Ähnliches gilt für gentechnisch hergestelltes PP4, das aus E.coli- bzw. Animalzellkulturen in biologisch aktiver Form gewonnen werden kann. Ein schnelles und mit möglichst geringen Verlusten verbundenes Verfahren zur Reinigung ist daher erforderlich.

Der Einsatz monoklonaler Antikörper zu Reinigungszwecken ist bereits für einige andere Proteine beschrieben und etabliert. Entscheidend für die Ausbeute und die Nativität des gereinigten Proteins ist, unter welchen Bedingungen bzw. mit welchen Agenzien das an immobilisierte monoklonale Antikörper gebundene Protein eluiert werden kann. Häufig kann die Bindung Dissoziation der zwischen monoklonalem Antikörper und Antigen nur durch extreme pH-Werte oder chaotrope Agenzien erreicht werden, was häufig zur Denaturierung des Proteins führt. Aus diesem Grund ist nicht jeder monoklonale Antikörper zur schonenden und schnellen Reinigung geeignet.

Aufgabe der Erfindung war es daher, monoklonale Antikörper gegen PP4 zu entwickeln, die, an geeignete Träger gebunden, eine schonende und effiziente Reinigung von PP4 aus verschiedenen Ausgangsmaterialien erlauben.

Gegenstand der Erfindung sind monoklonale Antikörper gegen PP4, ein Verfahren zu ihrer Herstellung und ihre Verwendung vorzugsweise zur Reinigung oder Diagnose von PP4.

Überraschenderweise wurden monoklonale Antikörper gefunden, die das Annexin PP4 nur in Gegenwart von zweiwertigen Kationen, vorzugsweise Calcium binden. Lösungen, die kein Calcium oder die chelatbildende Reagenzien enthalten, sind in der Lage, PP4 in biologisch aktiver Form mit hoher Ausbeute und Reinheit vom Antikörper zu desorbieren. Solche monoklonalen Antikörper, gebunden an geeignete Trägermaterialien, sind für die Reinigung von PP4 aus humanen Zellen bzw. Organen in gleicher Weise gut geeignet wie für die Reinigung eines rekombinanten PP4 aus E.coli- oder Animalzellkulturen. Solche Antikörper können ebenso für diagnostische Zwecke Verwendung finden.

Wie gezeigt wurde, kann PP4 als ein Marker für bestimmte Krankheiten angesehen werden. Normalerweise ist PP4 in humanen Körperflüssigkeiten, wie z.B. Plasma nur in sehr geringen Konzentrationen (0 - 10 ng/ml) nachweisbar. Bei bestimmten Krankheiten, z.B. einigen Tumoren, ist die Konzentration dieses Proteins signifikant erhöht und kann als ein Parameter für eine schnelle Diagnostik verwendet werden. Dies schließt sowohl die quantitativen Bestimmungen von PP4 mit Hilfe von monoklonalen Antikörpern in Körperflüssigkeiten, z.B. mit Hilfe von Radioimmunoassay oder Enzymimmunoassay ein, als auch die qualitative oder quantitative Bestimmung an Zelloberflächen, in Gewebeextrakten oder an histologischen Präparaten wie Gewebeschnitten.

Für diese Zwecke geeignete Antikörper können auf folgende Weise hergestellt werden:

## Gewinnung Antikörper-produzierender Zellen

Säugetiere, beispielsweise BALB/c-Mäuse, werden durch subkutane Injektion einer Emulsion von 50 μg PP4 (gereinigt aus humaner Placenta wie in Beispiel 1 beschrieben) in komplettem Freund'schem Adjuvans immunisiert (Tag 1). An den Tagen 28 und 56 wurden je 50 μg PP4, emulgiert in inkomplettem Freund'schem Adjuvans ebenfalls subkutan injiziert. Am Tag 92 folgt eine intraperitoneale Injektion von 100 μg PP4 in 0.5 ml physiologischer Kochsalzlösung. Am Tag 95 werden nach Entnahme der Milz durch mechanische Desintegration Lymphozyten gewonnen.

## Fusion von Lymphozyten mit Myelomzellen

Hybridomazellen werden nach Standardverfahren (Köhler und Milstein, 1975; Nature 256:495-497) gewonnen: Beispielsweise wird die Myelomzellinie SP2/0-Ag14 in Dulbecco's Modified Eagle's Medium (DMEM) mit 10 % fetalem Kälberserum (FKS) kultiviert. Für eine Fusion zur Erzeugung von Hybridomazellen werden typischerweise die Milzzellen einer Maus (ca. $10^8$) mit $5 \times 10^7$ Myelomzellen gemischt, in serumfreiem DMEM gewaschen und abzentrifugiert. Nach vollständiger Entfernung des Überstands werden dem Zellpellet 0.5 ml einer 50 %igen Lösung von Polyethylenglykol 4000 in DMEM innerhalb einer Minute zugetropft. Die Suspension wird 90 Sekunden bei 37 °c inkubiert und anschließend durch Zugabe von 7.5 ml DMEM über einen Zeitraum von 5 Minuten verdünnt. Nach einer Inkubation von 10 Minuten bei Raumtemperatur wird mit DMEM auf 40 ml aufgefüllt, und die Zellen werden abzentrifugiert. Nach Absaugen des Überstands werden die Zellen in DMEM mit 20 % FKS resuspendiert und auf 6 Mikrotiterplatten ausgesät (200 μl pro Kavität). Die Selektion von Hybridomazellen erfolgt durch Zusatz von 13.6 mg/ml Hypoxanthin, 0.18 mg/ml Aminopterin, 3.9 mg/ml Thymidin (HAT-Medium). Verbrauchtes Medium wird in Abständen von 3 - 4 Tagen durch frisches ersetzt, nach 10 Tagen wird HAT-Medium durch HT-Medium ersetzt.

**Biotinylierung von PP4**

Für den Test auf PP4-spezifische Antikörper wird biotinyliertes PP4 benötigt. Dieses kann auf folgende Weise hergestellt werden: PP4 wird gegen 0.1 M $NaHCO_3$, pH 8.0 dialysiert. Der Lösung werden 0.1 mg D-Biotin-N-hydroxysuccinimidester (0.1 %ige Lösung in Dimethylsulfoxid) pro Milligramm Protein zugesetzt. Das Reaktionsgemisch wird 4 h bei Raumtemperatur inkubiert und anschließend gegen 0.01 M $Na_2HPO_4$, 0.01 M $NaH_2PO_4$, pH 7.2, 0.15 M NaCl dialysiert.

**Test auf PP4-Antikörper**

14 Tage nach der Fusion werden die Zellkulturüberstände der fusionierten Zellen mittels Enzymimmunoassay auf Antikörper gegen PP4 getestet: Polystyrol-Mikrotestplatten werden mit 0.5 μg/ml Ziegeanti-Maus-IgG in 0.1 M $NaHCO_3$, pH 9.6, inkubiert (24 h, 4 °C). Anschließend werden Zellkulturüberstände appliziert (2 h, 37 °C), gefolgt von einer Inkubation mit biotinyliertem PP4 (1 μg/ml). Danach folgt eine Inkubation von 30 min bei 37 °C mit einem Komplex aus Avidin und biotinylierter Peroxidase (jeweils 1 μg/ml). Als Substrat wird eine Lösung von 0.1 % (Gew./Vol.) 2,2'-Azino-di-(3-ethylbenzthiazolinsulfonat-6) und 0.012 % (Vol./Vol.) $H_2O_2$ in 0.1 M Citronensäure, 0.1 M $Na_2HPO_4$, pH 4.5 verwendet. Nach einer Inkubation von 30 min bei 37 °C wird die Absorption bei 405 nm gemessen. Zwischen den einzelnen Inkubationsschritten wurde mit 0.01 M $Na_2HPO_4$, 0.01 M $NaH_2PO_4$, pH 7.2, 0.15 M NaCl, 0.05 % Tween 20 (PBS/Tween) gewaschen. Alle Reagenzien werden in 0.02 M Tris/HCl, pH 7.4, 0.15 M NaCl, 0.02 M $CaCl_2$, 2 % Rinderserumalbumin (TBS/RSA) verdünnt. Parallel dazu wird der Enzymimmunoassay mit denselben Zellkulturüberständen durchgeführt, wobei jedoch das Calciumchlorid im Verdünnungspuffer durch 0.02 M EDTA ersetzt wird. Antikörper, die PP4 nur in Gegenwart von $CaCl_2$, nicht aber von EDTA binden, werden selektiert. Diese sind zur Reinigung von PP4 mittels Immunaffinitätschromatographie besonders geeignet.

**Klonierung von Antikörper-produzierenden Zellinien**

Zellinien, die im Test auf PP4-Antikörper in Gegenwart von EDTA, nicht jedoch in Gegenwart von Calcium positiv reagieren, werden nach der Methode der limitierenden Verdünnung kloniert. Dazu werden ca. 60 Zellen in 20 ml DMEM mit 20 % FKS und 5 % Human Endothelial Culture Supernatant (Costar) auf 96 Kavitäten einer Zellkulturplatte verteilt. Einzelklone werden mikroskopisch identifiziert und auf Antikörperproduktion getestet. Die Klonierung wird zweimal wiederholt.

**Reinigung von monoklonalen Antikörpern**

Klonale Zellinien werden zur Produktion von Antikörpern in Roller-Flaschen überführt und in Iscove's Modified Dulbecco's Medium kultiviert. Zellüberstand wird durch Zentrifugation gewonnen und mit Hilfe von Ultrafiltration etwa 10fach konzentriert. Das Konzentrat wird über Protein A-$^R$Sepharose CL-4B (Pharmacia) gegeben, gebundenes IgG wird mit 0.2 M Glycin/HCl, pH 3.0 eluiert. Die proteinhaltigen Fraktionen werden gegen 0.1 M Citrat, pH 6.5, dialysiert und mit Ultrafiltration auf ca. 5 mg/ml konzentriert. Dabei wird beispielsweise ein Antikörper vom Subtyp kappa, IgG1 erhalten, der in der isoelektrischen Fokussierung in mehreren Banden im pH-Bereich 6.2 - 6.5 erscheint, und an den PP4 in Gegenwart von Calciumionen bindet.

Geeignete Immunaffinitätsgele können auf folgende Weise hergestellt werden: Einer der monoklonalen Antikörper, deren Herstellung vorstehend beschrieben ist, wird an einem geeigneten unlöslichen Trägermaterial (z.B. Agarose, Cellulose, Polyacrylamid und deren Derivate) mittels dem Fachmann vertrauten Verfahren (z.B. Aktivierung durch Bromcyan, Epoxide oder Carbodiimide) immobilisiert. In einer bevorzugten Verfahrensweise können solche monoklonalen Antikörper an bromcyan-aktivierte [R]Sepharose 4B (Pharmacia) nach den Angaben des Herstellers gekoppelt werden. Beispielsweise können 5 mg Antikörper pro 1 ml Gel gebunden werden. Diese Gele können dann zur Immunaffinitätschromatographie von PP4 verwendet werden.

Die folgenden Beispiele erläutern die Erfindung:

**Beispiel 1**

**Reinigung von PP4 aus humaner Placenta und Bestimmung der biologischen Aktivität**

36 kg humane Placenta wurden zerkleinert, mit 0.15 M NaCl mehrmals gewaschen und anschließend lyophilisiert. Das Lyophilisat (3 kg) wurde mit 50 1 0.02 M Tris/HCl, pH 7.5, 0.15 M NaCl, 0.1 M tri-Natrium-Citrat extrahiert, mit Ammoniumsulfat (33 % Sättigung) versetzt und mit 4 l Phenyl-[R]Sepharose im Batch-Verfahren inkubiert. Nach Waschen des Harzes wurde PP4 mit Wasser im Stoß eluiert, durch Zusatz von Ammoniumsulfat auf 80 % Sättigung gefällt, durch Zentrifugation pelletiert, in 0.02 M Tris/HCl, pH 8.0 (Puffer A) aufgenommen und gegen denselben Puffer dialysiert. Nach Zusatz von $CaCl_2$ bis zu einer Endkonzentration von 0.005 M wurde das Dialysat im Batch-Verfahren mit 1 l einer Heparin-[R]Sepharose versetzt, 60 min bei Raumtemperatur gerührt und die überstehende Lösung abgetrennt. Das Adsorbens wurde danach mit Puffer A unter Zusatz von 0.005 M $CaCl_2$ gewaschen. Die Elution von PP4 erfolgte durch einen linearen Gradienten von 0 - 0.5 M NaCl in calciumhaltigem Puffer A. Nach Dialyse der PP4-haltigen Fraktionen wurde EDTA bis zu einer Endkonzentration von 0.001 M zugesetzt und das Dialysat mit 200 ml einer Heparin-[R]Sepharose - äquilibriert in Puffer A mit 0.001 M EDTA - in Kontakt gebracht, für 60 min bei Raumtemperatur gerührt, und die überstehende, PP4-haltige Lösung abgetrennt. Gegebenenfalls wurde die PP4-haltige Lösung noch mit einer DEAE-[R]Sepharose in Kontakt gebracht und das Protein mittels eines linearen NaCl-Gradienten eluiert. Das auf diese Weise gereinigte PP4 erschien in der SDS-Polyacrylamid-Gelelektrophorese (PAGE) als eine Bande mit einem Molekulargewicht von 33 kDa. PP4 zeigte keine Kreuzreaktionen mit polyklonalen Antikörpern gegen fünf andere Annexine.

Die antikoagulatorische Aktivität von PPA wurde in einem modifizierten Thromboplastinzeit-Test überprüft: 50 $\mu$l Citrat-Plasma (Standard-Humanplasma) wurden mit 150 $\mu$l einer Lösung aus 0.05 M Tris/HCl, pH 7.5, 0.15 M NaCl, sowie 25 $\mu$l Puffer oder Probe und 25 $\mu$l Calcium-freiem Thromboplastin versetzt. Dieser Ansatz wurde für 3 min bei 37 °c inkubiert. Die Gerinnung wurde durch Zugabe von 25 $\mu$l einer Lösung aus 0.02 M $CaCl_2$ initiiert. Die Gerinnungszeit wurde mit einem Koagulometer nach Schnitger und Gross ermittelt. Eine Eichgerade wurde mittels des, wie oben beschrieben, aus Placenta gereinigten PPA erstellt.

**Beispiel 2**

**Reinigung von PP4 aus humaner Placenta mittels Immunaffinitätschromatographie**

Humane, reife Placenta wurde, wie in Beispiel 1 beschrieben gewaschen, lyophilisiert und mit Citrat-Puffer extrahiert. Dieser Extrakt wurde gegen 0.02 M Tris/HCl, pH 7.5 dialysiert. Eine Probe des Dialysates, welche 4.5 mg PP4 enthielt (bestimmt mit dem in Beispiel 4 beschriebenen Enzymimmunoassay), wurde mit $CaCl_2$ versetzt (Endkonzentration 0.02 M), und über ein Affinitätsgel (20 mg monoklonaler Antikörper gekoppelt an 4 ml CNBr-aktivierter [R]Sepharose 4B) gepumpt. Anschließend wurde mit 100 ml TBS/Calcium (0.02 M Tris, 0.15 M NaCl, 0.02 M $CaCl_2$, pH 7.4) nicht gebundenes Protein vom Gel gewaschen. Danach wurde mit 30 ml TBS ohne Calcium gespült, wobei PP4 vom Antikörper eluiert wurde. Die Ausbeute an Antigen und Aktivität bei der Immunaffinitätschromatographie betrug 92 bzw. 90 %. In der SDS-PAGE erschien PP4 als eine Bande mit einem Molekulargewicht von 33 kDa.

**Beispiel 3**

**Reinigung von rekombinantem PP4 aus E.coli mittels Immunaffinitätschromatographie**

Die für PP4 codierende cDNA, isoliert und charakterisiert aus einer humanen Placenta-Genbank, wurde kloniert und für die Expression in E.coli mittels des Vektors pTrc99A verwendet. Die Expression von rekombinantem PP4 (r-PP4) erfolgte durch Fermentation des E.coli-Stammes W31101acIQ. Die Fermentation von E.coli erfolgte nach dem Fachmann bekannten Verfahrensweisen. Nach Beendigung der Fermentation wurden die Zellen durch Zentrifugation geerntet. Das Pellet wurde in 0.02 M Tris/HCl, pH 7.5, und 0.01 M EDTA resuspendiert und die Zellen mittels einer "French Press" lysiert. Zellbruchstücke wurden durch Zentrifugation entfernt und der Überstand durch Filtration durch einen 0.45 µm-Filter nachgereinigt. Die r-PP4 enthaltende Losung wurde mit [R]Triton X-100 versetzt und gegen einen Puffer aus 0.02 M Tris/HCl, pH 7.5, dialysiert. Einer Probe des Dialysates, welche 3.8 mg PP4 enthielt (bestimmt mit dem in Beispiel 4 beschriebenen Enzymimmunoassay), wurde CaCl₂ zugesetzt (Endkonzentration 0.02 M), entstehender Niederschlag wurde abzentrifugiert, und der Überstand wurde wie in Beispiel 2 beschrieben durch Immunaffinitätschromatographie gereinigt. Die Ausbeute an PP4-Antigen und -Aktivität (bestimmt mit dem in Beispiel 4 beschriebenen Enzymimmunoassay bzw. mit dem in Beispiel 1 beschriebenen Aktivitätstest) betrug 94 bzw. 93 %. Das Protein erschien im SDS-Gel homogen mit einem Molekulargewicht von 33 kDa.

**Beispiel 4**

**Sandwich-ELISA zur Quantifizierung von PP4-Antigen**

Zur Quantifizierung von PP4 in biologischen Flüssigkeiten wurde ein Sandwich-ELISA entwickelt: Mikrotiterplatten wurden mit polyklonalen Antikörpern gegen PP4 (1 - 20 µg/ ml) beschichtet. Die PP4 enthaltende Probe (z.B. Placenta-Gewebeextrakte, E.coli-Lysate oder Plasma) wurde auf den Antikörper-beschichteten Platten inkubiert. Anschließend wurde mit einem biotinylierten monoklonalen Antikörper gegen PP4(2 µg/ml) inkubiert, gefolgt von einem Komplex aus Avidin und biotinylierter Peroxidase. Die Durchführung des Enzymimmunoassays und die Detektion der Peroxidaseaktivität erfolgte wie unter "Test auf PP4-Antikörper" beschrieben. Eine Eich- gerade wurde mittels des nach Beispiel 1 aus Placenta gereinigten PP4 erstellt. PP4 konnte in einem Konzentrationsbereich zwischen 125 ng/ml und 2 ng/ml bestimmt werden (Tabelle 1).

## Tabelle 1

| Konzentration PP4 (ng/ml) | Absorption 405 nm |
|---|---|
| 125 | 1.601 |
| 62.5 | 1.393 |
| 31.25 | 1.067 |
| 15.63 | 0.812 |
| 7.82 | 0.597 |
| 3.91 | 0.298 |
| 1.96 | 0.076 |

**Patentansprüche**

1. Monoklonale Antikörper gegen PP4.

2. Monoklonaler Antikörper nach Anspruch 1, dadurch gekennzeichnet, daß er nur in Gegenwart von zweiwertigen Metallionen an PP4 bindet.

3. Monoklonaler Antikörper nach Anspruch 1, dadurch gekennzeichnet, daß er nur in Gegenwart von Calciumionen an PP4 bindet.

4. Verfahren zur Herstellung eines monoklonalen Antikörpers nach Anspruch 1, dadurch gekennzeichnet, daß PP4 in ein Säugetier injiziert, aus einem lymphatischen Organ dieses Tieres Lymphozyten gewonnen, diese mit Zellen einer Myelomzellinie fusioniert, die gebildeten Hybridome kultiviert, die Zellüberstände auf mit PP4 reagierende Antikörper geprüft, Hybridome kloniert, erhaltene Klone kultiviert und aus den Kulturen monoklonale Antikörper gegen PP4 gewonnen werden.

5. Verfahren nach Anspruch 4, dadurch gekennzeichnet, daß ein monoklonalen Antikörper gewonnen wird, der nur in Gegenwart von zweiwertigen Metallionen an PP4 bindet.

6. Verwendung eines monoklonalen Antikörpers gegen PP4 zu Reinigung von PP4.

7. Verwendung nach Anspruch 6, dadurch gekennzeichnet, daß der Antikörper an ein unlösliches Material gebunden ist.

8. Verwendung eines monoklonalen Antikörpers gegen PP4 als Diagnostikum oder in einem diagnostischen Verfahren.

9. Verfahren zu Reinigung von PP4, dadurch gekennzeichnet, daß ein monoklonaler Antikörper gegen PP4 an ein für ein chromatographisches Reinigungsverfahren geeignetes unlösliches Material gebunden, PP4 aus einer Lösung an den gebundenen Antikörper adsorbiert und PP4 eluiert wird.

10. Verfahren nach Anspruch 9, dadurch gekennzeichnet, daß der monoklonale Antikörper einer ist, an den PP4 nur in Gegenwart von zweiwertigen Metallionen bindet, und daß mit einer Lösung, die solche Metallionen nicht oder in komplexierter Form enthält, PP4 eluiert wird.

**Patentansprüche für folgende Vertragsstaaten: ES und GR**

1. Verfahren zur Herstellung eines monoklonalen Antikörpers gegen PP4, dadurch gekennzeichnet, daß PP4 in ein Säugetier injiziert, aus einem lymphatischen Organ dieses Tieres Lymphozyten gewonnen, diese mit Zellen einer Myelomzellinie fusioniert, die gebildeten Hybridome kultiviert, die Zellüberstände auf mit PP4 reagierende Antikörper geprüft, Hybridome kloniert, erhaltene Klone kultiviert und aus den Kulturen monoklonale Antikörper gegen PP4 gewonnen werden.

2. Verfahren nach Anspruch 1, dadurch gekennzeichnet, daß ein monoklonaler Antikörper gewonnen wird, der nur in Gegenwart von zweiwertigen Metallionen an PP4 bindet.

3. Verfahren nach Anspruch 1, dadurch gekennzeichnet, daß ein monoklonaler Antikörper gewonnen wird, der nur in Gegenwart von Calciumionen an PP4 bindet.

4. Verfahren zur Reinigung von PP4, dadurch gekennzeichnet, daß ein monoklonaler Antikörper gegen PP4 an ein für ein chromatographisches Reinigungsverfahren geeignetes unlösliches Material gebunden, PP4 aus einer Lösung an den gebundenen Antikörper adsorbiert und PP4 eluiert wird.

5. Verfahren nach Anspruch 4, dadurch gekennzeichnet, daß der monoklonale Antikörper einer ist, an den PP4 nur in Gegenwart von zweiwertigen Metallionen bindet, und daß mit einer Lösung, die solche Metallionen nicht oder in komplexierter Form enthält, PP4 eluiert wird.

**Europäisches Patentamt**

**EUROPÄISCHER RECHERCHENBERICHT**

Nummer der Anmeldung

## EINSCHLÄGIGE DOKUMENTE

EP 91105971.5

| Kategorie | Kennzeichnung des Dokuments mit Angabe, soweit erforderlich, der maßgeblichen Teile | Betrifft Anspruch | KLASSIFIKATION DER ANMELDUNG (Int. Cl.⁵) |
|---|---|---|---|
| X | EP - A1 - 0 318 703 (BEHRINGWERKE) * Ansprüche 9,12,13 * | 1,4, 6-9 | C 12 P 21/08 C 07 K 15/06 C 07 K 3/18 C 12 N 5/12 G 01 N 33/577 |
| X | EP - A2 - 0 315 081 (BEHRINGWERKE) * Ansprüche 11,13 * | 1,4, 6-9 | |
| A | DE - A1 - 3 810 331 (BOEHRINGER INGELHEIM INTERNATIONAL GMBH) * Ansprüche 4,7,8 * | 1,4, 6-9 | |
| A | EP - A2 - 0 336 441 (KOWA COMPANY, LTD.) * Ansprüche 1-4 * | 1,4, 6-9 | |
| A | EP - A2 - 0 267 601 (KOWA CO. LTD.) * Ansprüche 1-4 * | 1,4, 6-9 | |
| A | CHEMICAL ABSTRACTS, Band 108, Nr. 13, 28. März 1988, Columbus, Ohio, USA R. BUETZON et al. "Monoclonal antibodies reacting with placental protein 5 : use in radioimmunoassay, Western blot analysis, and immunochemistry" Seite 460, Zusammenfassung-Nr. 110 504y & J.Lab.Clin.Med. 1988,111(2) 249-56 (Eng.) ---- | 1,4 | |

| | | | RECHERCHIERTE SACHGEBIETE (Int. Cl.⁵) |
|---|---|---|---|
| | | | C 12 P C 07 K C 12 N G 01 N |

Der vorliegende Recherchenbericht wurde für alle Patentansprüche erstellt.

| Recherchenort | Abschlußdatum der Recherche | Prüfer |
|---|---|---|
| WIEN | 09-07-1991 | AUGUSTIN |